# EUROPEAN PATENT SPECIFICATION

(11) **EP 2 083 853 B1**
(45) Date of publication and mention of the grant of the patent: **12.06.2013**
(21) Application number: 07859250.8
(22) Date of filing: 12.10.2007
(51) Int. Cl.: A61K 39/02

(54) **SHIGELLA IPAD PROTEIN AND ITS USE AS A VACCINE AGAINST SHIGELLA INFECTION**
SHIGELLA-IpaD-PROTEIN UND SEINE VERWENDUNG ALS IMPFSTOFF GEGEN SHIGELLEN-INFEKTION
PROTÉINE IPAD DE SHIGELLA ET SON UTILISATION EN TANT QUE VACCIN CONTRE UNE INFECTION À SHIGELLA

(30) Priority: 12.10.2006 CA 2563214
(43) Date of publication of application: 05.08.2009
(73) Proprietor: Institut Pasteur, 75724 Paris Cédex 15 (FR); Institut National de la Santé et de la Recherche Médicale, 75654 Paris Cedex 13 (FR); Université Libre de Bruxelles, 1050 Bruxelles (BE); Rijksuniversiteit Groningen, 9712 CP Groningen (NL)
(72) Inventor: ALLAOUI, Adbelmounaaïm, B-1070 Bruxelles (BE); SANSONETTI, Philippe, F-75014 Paris (FR); SANI, Musa, 1066 NJ Amsterdam (NL); BOTTEAUX, Anne, 1502 Lembeek (BE); PARSOT, Claude, F-75015 Paris (FR); BOEKEMA, Egbert, J., NL-9742 EV Groningen (NL)
(74) Representative: Desaix, Anne
(86) International application number: PCT/IB2007/004192
(87) International publication number: WO 2008/044149

(56) References cited:
- WO-A-00/18355
- TURBYFILL K ROSS ET AL: "Isolation and characterization of a Shigella flexneri invasin complex subunit vaccine" INFECTION AND IMMUNITY, AMERICAN SOCIETY FOR MICROBIOLOGY. WASHINGTON, vol. 68, no. 12, 1 December 2000 (2000-12-01), pages 6624-6632, XP002460410 ISSN: 0019-9567
- OAKS EDWIN V ET AL: "Antibody response of monkeys to invasion plasmid antigen D after infection with Shigella spp" CLINICAL AND DIAGNOSTIC LABORATORY IMMUNOLOGY, vol. 3, no. 2, 1996, pages 242-245, XP002479749 ISSN: 1071-412X
- TURBYFILL K R ET AL: "Identification of epitope and surface-exposed domains of Shigella flexneri invasion plasmid antigen D ( IpaD )" INFECTION AND IMMUNITY, AMERICAN SOCIETY FOR MICROBIOLOGY. WASHINGTON, vol. 66, no. 5, 1 May 1998 (1998-05-01), pages 1999-2006, XP002141130 ISSN: 0019-9567
- MENARD R ET AL: "The secreted Ipa complex of Shigella flexneri promotes entry into mammalian cells" PROCEEDINGS OF THE NATIONAL ACADEMY OF SCIENCES OF THE UNITED STATES OF AMERICA 1996 US, vol. 93, no. 3, 1996, pages 1254-1258, XP002479750 ISSN: 0027-8424
- SANI MUSA ET AL: "IpaD is localized at the tip of the Shigella flexneri type III secretion apparatus." BIOCHIMICA ET BIOPHYSICA ACTA FEB 2007, vol. 1770, no. 2, February 2007 (2007-02), pages 307-311, XP002479751 ISSN: 0006-3002

## Description

### FIELD OF THE INVENTION

The present invention relates to compositions and methods for use in blocking entry of Shigella into a cell of an animal, to therefore providing protection against, or reduce the severity of Shigella infections. More particularly it relates to the use of the IpaD protein obtained from natural sources and/or through synthesis or recombinant technology, and conjugates thereof to induce neutralizing antibodies having protective activity against several serotypes of *Shigella,* in particular S. *flexneri.* The composition of the invention is useful to prevent and/or treat shigellosis caused by a bacterium of the Shigella family.

### BACKGROUND OF THE INVENTION

Many Gram-negative pathogenic bacteria use a type three secretion (T3S) system to interact with cells of their host. Each T3S system consists of a secretion apparatus (T3SA) that spans the bacterial envelope and extends on the bacterial surface, translocators that transit through the T3SA and insert into the membrane of the host cell where they form a pore, effectors that transit through the T3SA and the translocator pore to reach the cell cytoplasm, specific chaperones that associate with translocators and effectors in the bacterial cytoplasm and transcriptional regulators. Approximately 15 proteins are required for assembly of the T3SA.

Bacteria belonging to the *Shigella* family are the causative agents of bacillary dysentery in humans [1]. Genes required for entry of bacteria into epithelial cells and inducing apoptosis in macrophages are clustered in a 30-kb region, designated the entry region, of a 220-kb virulence plasmid. The entry region contains *mxi* and *spa* genes encoding components of the T3SA, the *ipaA, B*, *C* and *D, ipgB1, ipgD* and *icsB* genes encoding proteins that transit through the T3SA, the *ipgA, ipgC, ipgE* and *spa15* genes encoding chaperones, and the *virB* and *mxiE* genes encoding transcriptional regulators [2].

The T3SA, which is weakly active in bacteria growing in broth, is activated upon contact of bacteria with epithelial cells [3]. Inactivation of *ipaB, ipaC or ipaD,* as well as most *mxi* and *spa* genes, abolishes the ability of bacteria to enter epithelial cells, induce apoptosis in macrophages and express contact hemolytic activity. IpaB and IpaC contain hydrophobic segments and remained associated with the membrane of lyzed erythrocytes, suggesting that these two proteins are components of the *S. flexneri* transtocator. In addition, effector functions have been proposed for IpaB and IpaC [4,5,6,7,8]. Inactivation of *ipaB* and *ipaD,* but not *ipaC*, leads to a deregulated, i. e. constitutively active, T3SA, suggesting that IpaB and IpaD play a role in maintaining the T3SA inactive in the absence of inducers [9, 10]. A small proportion of IpaD is associated with the bacterial envelope [9, 11]. Picking and collaborators [12] reported that the role of IpaD in the control of the T3SA activity can be separated from its role in entry of bacteria into epithelial cells.

To get further insights on the structure of the needle complex, the inventors performed an immuno-electron microscopic analysis on bacteria treated with the cross-linking agent BS³, both on entire bacteria and on the mildly purified needle complex (NC). The inventors present evidence that IpaD is a component of the NC localized at the tip of the needle and that antibodies raised against IpaD have an inhibitory effect on entry of *S. flexneri* into epithelial cells.

### BRIEF DESCRIPTION OF THE FIGURES

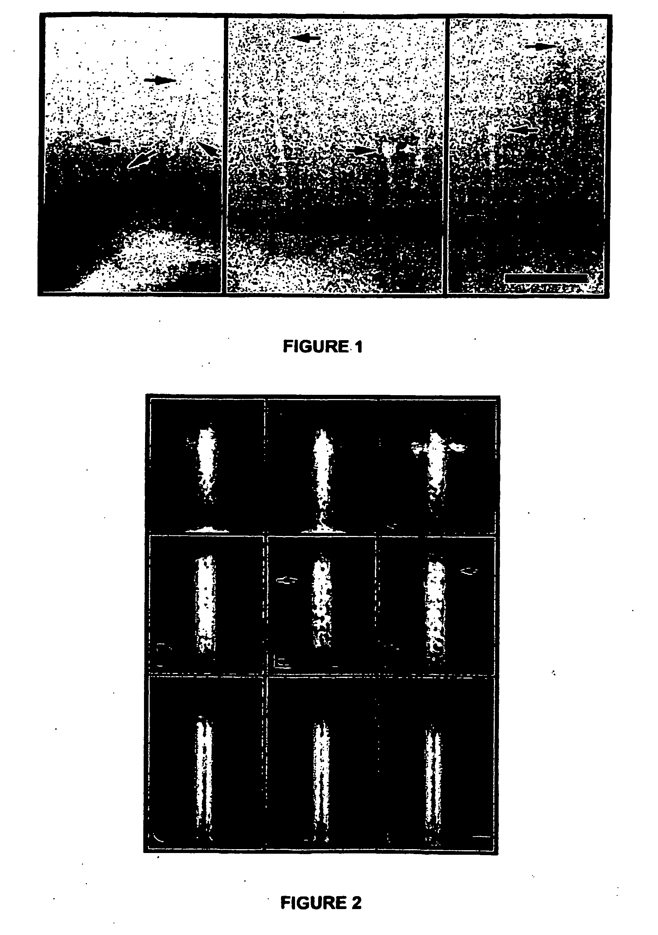

**Fig. 1**: Electron micrographs of negatively stained bacteria treated with BS³. Arrows indicate distinct densities at the tip of needles protruding from the bacterial surface. The bar represents 100 nm.
**Fig. 2**: Projection maps (class-sums) of the needle part of NCs obtained by single particle analysis. (A-B) maps of NCs isolated from wild-type, treated with BS³ (A-C); from wild-type without BS³ treatment (D-F) and from an *ipaD* mutant strain with BS³ treatment (G-I). The arrows point to remnant densities attached to needle parts prepared without BS³-treatment. The bar represents 10 nm.

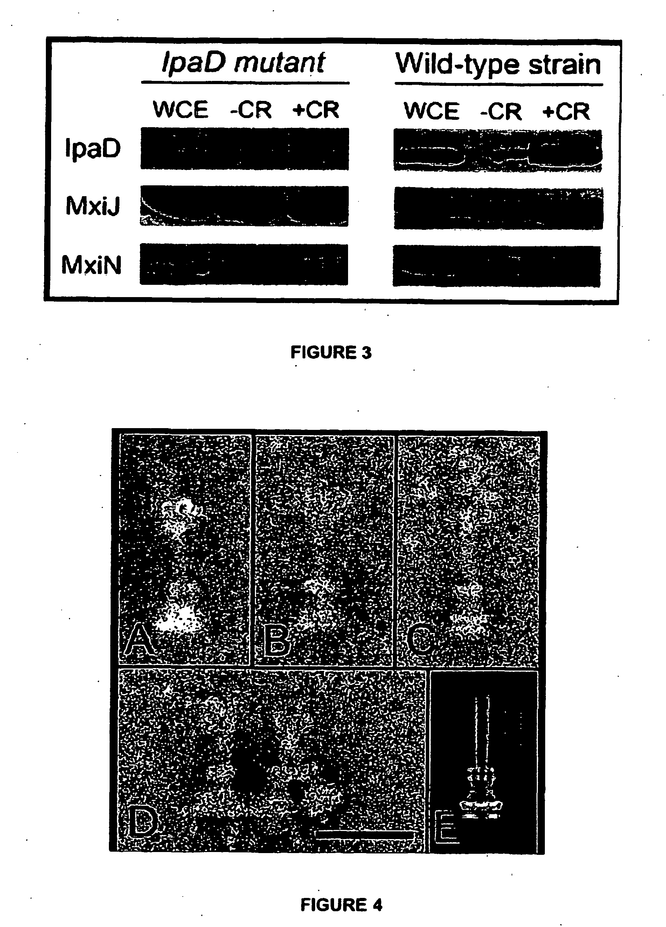

**Fig. 3**: Immunobloting analysis of purified NCs. Whole cell extracts (WCE) and cross linked (+CR) and non-cross linked (-CR) NCs purified from wild-type and IpaD deficient strains were analyzed by SDS-PAGE and immunoblotting using antibodies specific to MxiJ, MxiN, and IpaD. IpaD was only enriched to WCE level in NCs prepared from cross-linked wild-type bacteria. MxiJ is a positive control to demonstrate intact T3SA. MxiN, a cytoplasmic component of T3S, is a positive control to demonstrate contamination by non-bound cytoplasmic proteins.
**Fig. 4**: IpaD localization by immuno-electron microscopy. NCs purified from BS³ treated wild-type bacteria were incubated with anti-IpaD antibodies and negatively stained (A-D). The average image of 250 NCs purified from bacteria not treated with BS³ is shown in E for comparison. The bar represents 10 nm.

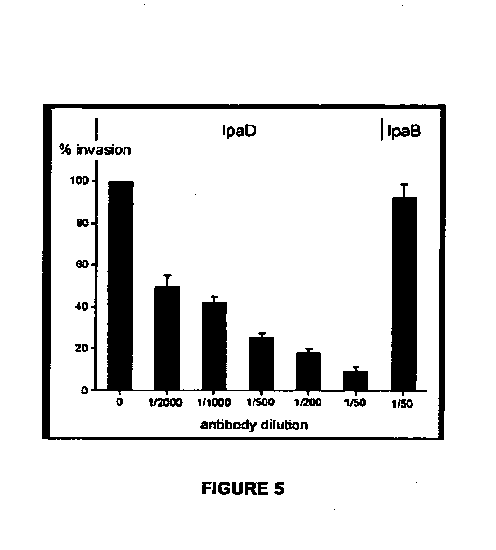

**Fig. 5**: Invasion assay of epithelial cells by wild-type *S. flexneri.* Bacteria were incubated with serial dilutions of the anti-IpaD or anti-IpaB polyclonal antibodies and intracellular, gentamycin-resistant bacteria were counted by plating cell lysates. The efficiency of entry in each condition is expressed with respect to that of the wild-type strain treated with PBS. The values are the means of at least three independent experiments, and the error bars indicate standard deviations.

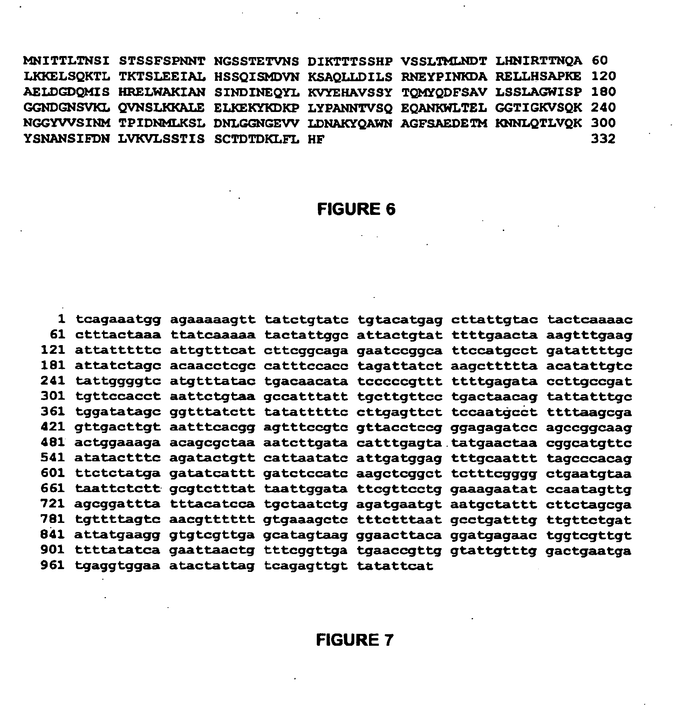

**Fig. 6**: Prior art amino acid sequence of the IpaD protein of a Shigella strain (GenBank accession number AL391753).
**Fig. 7**: Prior art nucleic acid sequence encoding the IpaD protein of Fig. 6 (GenBank accession number AL391753).

### DESCRIPTION OF THE INVENTION

The inventors have surprisingly found that IpaD-specific neutralizing antibodies can block the entry of Shigella into permissive cells, such as epithelial cells and that this neutralizing effect is observed on different Shigella serotypes. In this connection, the present invention specifically relates to the use of the IpaD protein, the polynucleotide encoding same or anti-IpaD neutralizing antibodies in the preparation of compositions and use of said compositions to elicit a cross protection against Shigella infection.

### Definitions

The terms "animal" or "host" refer to any animal susceptible or known to be infected by a *Shigella* strain, such as S. *flexneri.* Specifically, the animal consists of a human.

The term "permissive cell" refers to a cell that can be infected by a Shigella strain. For instance, such a cell may be, but not limited to an epithelial cell or cells of the immune systems, particularly those that are targets for Shigella invasion in the intestinal mucosal immune system, such as dendritic cells and monocytes/macrophages, but also B and T lymphocytes that may undergo injection of Shigella effectors through the Type III secretion system, even if this does not lead to their invasion.

The term "treating" refers to a process by which the symptoms of an infection or a disease associated with a *Shigella* strain are alleviated or completely eliminated. As used herein, the term "preventing" refers to a process by which symptoms of an infection or a disease associated with a *Shigella* strain are obstructed or delayed.

The term "protective response" means prevention of onset of a *Shigella* -associated disease or an infection caused by a *species* or lessening the severity of such a disease existing in an animal.

The expression "an acceptable carrier means a vehicle for containing the elements of the composition contemplated by the present invention that can be administered to an animal host without adverse effects. Suitable carriers known in the art include, but are not limited to, gold particles, sterile water, saline, glucose, dextrose, or buffered solutions. Carriers may include auxiliary agents including, but not limited to, diluents, stabilizers (i.e., sugars and amino acids), preservatives, wetting agents, emulsifying agents, pH buffering agents, viscosity enhancing additives, colors and the like.

A "functional derivative", as is generally understood and used herein, refers to a protein/peptide sequence that possesses a functional biological activity that is substantially similar to the biological activity of the whole IpaD protein/peptide sequence. In other words, it refers to a polypeptide or fragment(s) thereof that substantially retain(s) the capacity of eliciting the production of anti-IpaD neutralizing antibodies against a S. strain infection when said functional polypeptide derivative is administered to an animal.

A "functional fragment", as is generally understood and used herein, refers to a nucleic acid sequence that encodes for a functional biological activity that is substantially similar to the biological activity of the whole IpaD nucleic acid sequence. In other words, and within the context of the present invention, it refers to a nucleic acid or fragment(s) thereof that substantially retains the capacity of encoding a IpaD polypeptide/protein which elicits the production of anti-IpaD neutralizing antibodies against a *Shigella* strain infection when administered to an animal.

The term "Shigella serotype" refers to the four groups of Shigella that are identified by a capital letter from A-D: Shigella dysenteriae (A) Shigella flexneri (B), Shigella boydii (C), and Shigella sonnei (D). They respectively comprise: Group A: 8 serotypes, Group B: 11 serotypes and subserotypes, Group C: 11 serotypes, Group D: 1 serotype. For instance, a Shigella serotype may be, but not limited to, Shigella flexneri 2a, 1b and 3a, Shigella dysenteriae 1 and Shigella sonnei.

By the term "neutralizing" or "blocking", it is referred to the ability of the anti-IpaD antibodies of the invention to specifically bind to the IpaD protein and to interfere with the biological function of the IpaD protein therefore blocking, for instance, the capacity of the bacterium to deliver its effectors of virulence to the target cells. In other words, such antibodies advantageously disarm the pathogen and make it both unable to cause lesions, and incapable to resist efficiently to host immune defences.

### Compositions and compounds of the invention

In one aspect, the invention provides a composition for use in the treatment and/or the prevention of a Shigella infection. The invention also provides a composition for use in blocking entry of at least one Shigella serotype into a permissive cell. These contemplated compositions of the invention comprise at least one of the following elements:
- an IpaD polypeptide or functional polypeptide derivative or a fragment thereof that retains the capacity of eliciting the production of anti-IpaD neutralizing antibodies against a Shigella strain when administered to an animal:;
- a polynucleotide encoding an IpaD polypeptide or a fragment thereof that retains the capacity of encoding a IpaD polypeptide which elicits the production of anti-IpaD neutralizing antibodies against a Shigella strain when administered to an animal: and/or;
- an anti-IpaD neutralizing antibody.

As one skilled in the art may appreciate, the compositions of the invention advantageously provide a cross-protection against more than one Shigella serotype when administered to a host. In other words, the compositions of the invention prevent or substantially reduce the entry into a permissive cell of more than one Shigella serotype.

The invention also concerns an IpaD polypeptide or a functional polypeptide derivative or a fraament thereof that retains the capacity of eliciting the production of anti-IpaD neutralizing antibodies against a Shigella strain when administered to an animal, or a polynucleotide encoding_ an IpaD polypeptide or a functional fragment thereof that retains the capacity of encoding a IpaD polypeptide which elicits the production of anti-IpaD neutralizing antibodies against a Shigella strain when administered to an animal: and/or an anti-IpaD neutralizing antibody, for use in the treatment and/or the prevention of a Shigella infection.

The IpaD polypeptide or functional derivative thereof contemplated by the present invention has for instance an amino acid sequence which is identical or substantially identical to the amino acid sequence having the GenBank accession number AL391753, and depicted herein as SEQ ID. NO:1 (see Fig. 6). In the case of a functional derivative of the IpaD polypeptide, one may use the plasmid pMal-IpaD deposited at the CNCM on October 10, 2007 under accession number 1-3839. This plasmid encodes a fragment of the IpaD protein starting at codon 130 of the IpaD protein.

By "substantially identical" when referring to an amino acid sequence, it will be understood that the polypeptide contemplated by the present invention has an amino acid sequence having at least 75% identity, or 85% identity or even 95% identity to part or all of the sequence shown in SEQ ID NO:1.

The polynucleotide or functional fragment thereof contemplated by the present invention codes for the IpaD polypeptide or a functional polypeptide derivative thereof as defined above. For instance, such a polynucleotide has a nucleotide or nucleic acid sequence which is identical or substantially identical to the nucleotide sequence having GenBank accession number AL391753 and depicted herein as SEQ ID NO:2 (see Fig. 7). In the case of a functional fragment of the IpaD polynucleotide, one may use the plasmid pMal-IpaD as defined above.

By "substantially identical" when referring to a nucleotide or polynucleotide sequence, it will be understood that the polynucleotide contemplated by the present invention has a nucleic acid sequence which is at least 65% identical, or 80% identical, or even 95% identical to part or all of the sequence shown in SEQ ID NO:2.

Techniques for determining nucleic acid and amino acid "sequence identity" also are known in the art. Typically, such techniques include determining the nucleotide sequence of the mRNA for a gene, the DNA sequence itself, and/or determining the amino acid sequence encoded thereby, and comparing these sequences to a second nucleotide or amino acid sequence. In general, "identity" refers to an exact nucleotide-to-nucleotide or amino acid-to-amino acid correspondence of two polynucleotides or polypeptide sequences, respectively. Two or more sequences (polynucleotide or amino acid) can be compared by determining their "percent identity." The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. The default parameters for this method are described in the Wisconsin Sequence Analysis Package Program Manual, Version 8 (1995) (available from Genetics Computer Group, Madison, Wis.). A preferred method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, Calif.). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix BLOSUM62; Descriptions=50 sequences; sort by=HIGH SCORE; Databases=non-redundant, GenBank+EMBL+DDBJ+PDB+GenBank CDS translations+Swiss protein+Spupdate+PIR.

The neutralizing antibodies contemplated by the invention which specifically bind to the IpaD protein may be prepared by a variety of methods known to one skilled in the art. For example, the IpaD polypeptide may be administered to an animal in order to induce the production of polyclonal antibodies. Alternatively, the anti-IpaD neutralizing antibodies used as described herein may be monoclonal antibodies, which are prepared using known hybridoma technologies (see, e.g., Hammerling et al., In Monoclonal Antibodies and T-Cell Hybridomas, Elsevier, NY, 1981). With respect to contemplated antibodies of the present invention, the term "specifically binds to" refers to antibodies that bind with a relatively high affinity to one or more epitopes of the IpaD polypeptide, but which do not substantially recognize and bind molecules other than the IpaD polypeptide. As used herein, the term "relatively high affinity" means a binding affinity between the antibody and the IpaD polypeptide of at least 10⁸ M⁻¹, and preferably of at least about 10⁷ M⁻¹ and even more preferably 10⁸ M⁻¹ to 10¹⁰ M⁻¹. Determination of such affinity is preferably conducted under standard competitive binding immunoassay conditions which are common knowledge to one skilled in the art.

It is understood that it is within one's knowledge in the field to screen and identify antibodies that neutralize the entry of Shigella into a cell.

In a preferred embodiment, the composition of the invention further comprises an adjuvant. As used herein, the term "adjuvant" means a substance added to the composition of the invention to increase the composition's immunogenicity. The mechanism of how an adjuvant operates is not entirely known. Some adjuvants are believed to enhance the immune response (humoral and/or cellular response) by slowly releasing the antigen, while other adjuvants are strongly immunogenic in their own right and are believed to function synergistically. Known adjuvants include, but are not limited to, oil and water emulsions (for example, complete Freund's adjuvant and incomplete Freund's adjuvant), Corytzebactei-ium parvuin, Quil A, cytokines such as IL12, Emulsigen-Plus®, Bacillus Calmette Guerin, aluminum hydroxide, glucan, dextran sulfate, iron oxide, sodium alginate, Bacto Adjuvant, certain synthetic polymers such as poly amino acids and co-polymers of amino acids, saponin, paraffin oil, and muramyl dipeptide. Adjuvants also encompass genetic adjuvants such as immunomodulatory molecules encoded in a co-inoculated DNA, or as CpG oligonucleotides. The coinoculated DNA can be in the same plasmid construct as the plasmid immunogen or in a separate DNA vector.

According a preferred embodiment of the invention, the compositions may further comprise a polyosidic antigen, such as those described in WO 2005/003995. For instance, contemplated polyosidic antigens may be, but not limited to synthetic polysaccharides corresponding to the O-side chains (constitutives of the serotypes) of strains of interest, particularly those serotypes whose high prevalence is preferably considered: *Shigella* flexneri.2a,- 1 b and 3a, Shigella dysenteriae 1 and Shigella sonnei. The contemplated polyosidic antigens may also be detoxified lipopolysaccharide (LPS) extracted from bacterial cultures of similar serotypes.

### Methods of treatment and compositions

The IpaD polypeptide or functional polypeptide derivatives, the polynucleotide or functional fragments encoding same and anti-IpaD neutralizing antibodies contemplated by the invention may be used in many ways in the treatment and/or prevention of *Shigella* infection, or in the blocking entry of Shigella strains into a permissive cell.

For instance, and according to an aspect of the invention, the IpaD polypeptide may be used as immunogens for the production of specific anti-IpaD neutralizing antibodies. As previously mentioned, suitable anti-IpaD neutralizing antibodies may be determined using appropriate known screening methods, for example by measuring the ability of a particular antibody to neutralize or block a *Shigella* strain to enter into a cell.

According to the disclosure, the polynucleotides encoding an Ipad polypeptide or polypeptide derivatives thereof may be used in a DNA immunization method so as to produce anti-IpaD neutralizing antibodies. That is, they can be incorporated into a vector which is replicable and expressible upon injection thereby producing the antigenic polypeptide *in vivo.* For example polynucleotides may be incorporated into a plasmid vector under the control of the CMV promoter which is functional in eukaryotic cells. Preferably the vector is injected intramuscularly.

The use of a polynucleotide of the invention in genetic immunization will preferably employ a suitable delivery method or system such as direct injection of plasmid DNA into muscles [Wolf et al. H M G (1992) 1: 363, Tumes et al., Vaccine (1999), 17 : 2089, Le et al., Vaccine (2000) 18 : 1893, Alves et al., Vaccine (2001)19 : 788], injection of plasmid DNA with or without adjuvants [Ulmer et al., Vaccine (1999) 18: 18, MacLaughlin et al., J. Control Release (1998) 56: 259, Hartikka et al., Gene Ther. (2000) 7:1171 -82, Benvenisty and Reshef, PNAS USA (1986) 83:9551, Singh et al., PNAS USA (2000) 97:811], targeting cells by delivery of DNA complexed with specific carriers [Wa et al., J Biol Chem (1989) 264:16985. Chaplin et al., Infect. Immun. (1999) 67:6434], injection of plasmid complexed or encapsulated in various forms of liposomes [Ishii et al., AIDS Research and Human Retroviruses (1997) 13:142, Perrie et al., Vaccine (2001) 19:3301], administration of DNA with different methods of bombardment [Tang et al.. Nature (1992) 356: 152, Eisenbraun et al. , DNA Cell Biol (1993) 12: 791, Chen et al. , Vaccine (2001) 19:2908], and administration of DNA with lived vectors [Tubulekas et al. , Gene (1997) 190:191, Pushko et al., Virology (1997) 239:389, Spreng et al. FEMS(2000) 27: 299. Dietrich et al., Vaccine (2001) 19:2506].

A further aspect of the disclosure is the use of the specific anti-IpaD neutralizing antibodies for passive immunization.

Yet, another aspect of the disclosure is to provide a method for treating and/or preventing a *Shigella* infection in an animal. The method of the invention comprises the step of administering to the animal a composition according to the invention.

Yet, a further aspect of the invention is to provide an anti-IpaD neutralizing antibody for use for blocking entry of at least one Shingella serotype into a permissive cell, wherein said anti-IpaD neutralizing antibody forms an immune complex by contacting said anti-IpaD neutralizing antibody with a Shigella strain capable of infecting said permissive cell, said immune complex preventing or substantially reducing entry of said Shigella strain in the permissive cell.

The amount of the components or the elements of the compositions of the invention is preferably a therapeutically effective amount. A therapeutically effective amount of the contemplated component is the amount necessary to allow the same to perform their immunological role (i.e. production of anti-IpaD neutralizing antibodies) without causing overly negative effects in the host to which the composition is administered. The exact amount of the components to be used and the composition to be administered will vary according to factors such as the type of condition being treated, the type and age of the animal to be treated, the mode of administration, as well as the other ingredients in the composition.

The compositions disclosed may be given to an animal through various routes of administration. For instance, the compositions may be administered in the form of sterile injectable preparations, such as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents.

They may be given parenterally, for example intravenously, intramuscularly or sub-cutaneously by injection, by infusion or *per os.* Suitable dosages will vary, depending upon factors such as the amount of each of the components in the compositions, the desired effect (short or long term), the route of administration, the age and the weight of the animal to be treated. Any other methods well known in the art may be used for administering the composition of the invention.

A further aspect of the invention is to provide kits for use blocking entry of at least one Shigella serotype into a permissive cell. Components may be compounds, reagents, containers and/or equipment. For example, one container within a kit may contain a monoclonal antibody or fragment thereof or polyclonal antibodies that specifically neutralize an IpaD protein. One or more additional containers may enclose elements, such as reagents or buffers, to be used in the assay. Kits contemplated by the present invention may comprise at least one IpaD polypeptide or a polynucleotide encoding same or an anti-IpaD neutralizing antibody as defined herein as described above, to direct a neutralizing immune response against the IpaD protein of Shigella.

The invention also relates to the polypeptide or a fragment thereof, or a polynucleotide or a fragment thereof, or an antibody as defined according to the invention for use in the preparation of a vaccine effective against several serotypes of Shigella.

The present invention will be more readily understood by referring to the following examples. These examples are illustrative of the wide range of applicability of the present invention and are not intended to limit its scope. Modifications and variations can be made therein without departing from the spirit and scope of the invention. Although any methods and materials similar or equivalent to those described herein can be used in the practice for testing of the present invention, preferred methods and materials are described hereinafter.

### EXAMPLES

Type III secretion (T3S) systems are used by numerous Gram-negative pathogenic bacteria to inject virulence proteins into animal and plant host cells. The core of the T3S apparatus, known as the needle complex, is composed of a basal body transversing both bacterial membranes and a needle protruding above the bacterial surface. In *Shigella flexneri,* IpaD is required to inhibit the activity of the T3S apparatus prior to contact of bacteria with host and has been proposed to assist translocation of bacterial proteins into host ceils. The inventors investigated the localization of IpaD by electron microscopy analysis of cross-linked bacteria and mildly purified needle complexes. This analysis revealed the presence of a distinct density at the needle tip. A combination of single particle analysis, immuno-labeling and biochemical analysis, demonstrated that IpaD forms part of the structure at the needle tip. Anti-IpaD antibodies were shown to block entry of bacteria into epithelial cells.

### General materials and methods

### Bacterial strains and growth media

Strains used in this study are the wild-type S. *flexneri* 5 strain M90T-Sm [13], its *ipaD* derivative SF622 [14]. Bacteria were grown in tryptic casein soy broth (TSB) (Sigma) at 37°C.

### Purification of needle complex (NC)

NCs were purified as described [15]. Bacteria in the exponential phase of growth in 1 I of TSB at 37°C were collected by centrifugation, resuspended in 25 ml of phosphate-buffered saline and incubated in the presence of 1 mM Bis (Sulfosuccinimidyl)suberate (BS³) for 30 min at 37°C. The mixture was supplemented with 100 mM Tris-HCl and incubated for 15 min at 37°C. BS³-treated cultures were harvested and resuspended in an ice-cold lysis buffer (0.5 M sucrose, 20 mM Tris-HCl [pH 7.5], 2 mM EDTA, 0.5 mg/ml lysozyme) supplemented with 1 mM phenylmethylsulfonyl fluoride and incubated for 45 min at 4°C and for 15 min at 37°C. Resulting spheroplasts were incubated with 0.01 % Triton X-100 for 30 min and treated with 4 mM MgCl₂ and 80 µg/ml DNAse (Sigma) for 20 min at 30°C. Debris were removed by centrifugation (20,000 g for 20 min at 4°C) and the membrane fraction was pelleted by centrifugation (110,000 g for 30 min at 4°C) and resuspended in TET buffer (20 mM Tris-HCl pH 7.5, 1 mM EDTA, 0.01% Triton X-100). Immunoblotting analysis was performed with antibodies raised against MxiJ, MxiN and IpaD as described [16].

### Electron microscopy and image analysis

Whole cells and samples of purified NCs were negatively stained with 2% uranyl acetate on glow discharged carbon-coated copper grids. Electron microscopy was performed on a Philips CM120FEG equipped with a field emission gun operated at 120 kV. Images were recorded with a 4000 SP 4K slow-scan CCD camera at 80,000 x magnification at a pixel size (after binning the images) of 3.75 A at the specimen level, with "GRACE" software for semi-automated specimen selection and data acquisition [17]. Single particle analysis including multi-reference and non-reference procedures, multivariate statistical analysis and classification was performed as described [15]. For immuno-labeling, purified NCs were incubated with affinity purified IpaD polyclonal antibodies (pAbs) at a final concentration of 0.132 ng/µl) for 1 hr at 20°C. Samples were stained with 2% uranyl acetate and observed as above.

### Invasion assay

Two ml of cultures of wild-type or *mxiD* strains in the exponential phase of growth (OD₆₀₀ₙₘ of 0.4) were incubated in the presence of anti-IpaD (dilution 1/2000 to 1/50) or anti-IpaB (1/50) antibodies for 1 h at 37°C and bacteria were centrifuged on plates containing 2 10⁵ Hela cells for 10 min at 2000 g. After 1 h incubation at 37°C, cells were washed three times with 2 ml EBSS and incubated during 1 h with 2 ml MEM milieu containing 50 µg/ml gentamycin. After three washes with 2 ml EBSS, plates were incubated with a solution of deoxycholate 0.5% for 15 min at 20°C and cell lysates were diluted and plated on agar plates for colony counting.

### Example 1: A distinctive structure at the tip of the T3SA needle

Protein purification procedures tend to select for most stable complexes that might not contain weakly associated subunits. The inventors recently showed that a Triton-X100 detergent concentration as low as 0.01% was sufficient to induce the release of NCs from the membrane (Sani et *al.,* 2006). To detect potentially labile subunits attached to the needle, the inventors performed a cross-linking step with BS³ on bacteria prior to any purification. Electron microscopy analysis indicated that, following BS³ treatment, most bacteria exhibited needle appendages with an additional density at the extremity of the tip (Fig. 1).

NCs were purified from BS³-treated-bacteria after detergent solubilization of membranes as described [18]. Preparations contained a sufficient number of NCs with the additional densities at the needle tip to perform a structural analysis. To calculate two-dimensional projection maps of isolated NCs, electron microscopy images were analysed by single particle analysis. The inventors selected several hundred images of NCs with a relatively straight and short needle appendage and a length dose to 45 nm. The averaged NCs clearly showed the presence of a density around the needle tip, as well as the upper part of the basal body (Fig. 2A and 2B; see also Fig. 4E for a total view of a NC). However, NCs appeared a bit blurred after averaging as a result of variations in the needle length. Sharper features at the tip of the needle portion were obtained when projections were aligned and classified after masking the basal part (Fig. 2C). Striking features of the average map of cross-linked particles are the presence of densities at either side of the needle tip. In contrast, average maps of particles prepared from the wild-type strain without cross-linking showed needles lacking most of these densities (Fig. 2D-F). Faint densities are visible in these samples at the same position where the strong densities were present in cross-linked preparations (arrows, Fig. 2E and 2F). These results suggest that, in the absence of cross-linking, most purified NCs lost the additional molecule(s) forming the density observed after cross-linking. To identify molecule(s) forming the density at the tip of the T3SA, the inventors performed similar experiments with an *ipaD* mutant lacking the IpaD expression. IpaD is required, together with IpaB, to maintain the T3SA inactive in the absence of inducers and a small proportion of IpaD is membrane associated [9]. NCs purified from the BS³-treated *ipaD* mutant did not exhibit densities at either side of the needle tip (Fig. 2G-I), suggesting that IpaD is part of or required for assembly of this structural element.

### Example 2: IpaD is present at the tip of the T3SA needle

To test whether IpaD constitutes the observed density, NCs purified from BS³-treated wild-type bacteria were analyzed by SDS-PAGE and immunoblotting (Fig. 3). IpaD was enriched in NCs prepared from cross-linked wild-type bacteria, as compared to NCs prepared from non-cross-linked bacteria (Fig. 3, right panel, right lane), though small amounts also co-purifiy in non-cross linked preparation and thus corroborate the faint densities at the needle tip for averages of non cross linked NCs. MxiJ that is a major NC component is present in similar amounts in all preparations (Fig. 3). Control experiments using antibodies recognizing cytoplasmic components of the T3S system, such as MxiN, did not reveal any contamination of NCs by intracellular components (Fig. 3), indicating that the presence of IpaD in the preparations was not due to a contamination by cytoplasmic proteins.

To confirm that densities detected at the tip of NCs contained IpaD. the inventors performed immuno-staining using an anti-IpaD serum. Antibodies specifically bound to the tip of the needle in NCs prepared from BS³ treated wild-type bacteria (Fig. 4A-D). Some needles were also observed to be associated by their tip, presumably as a result of the interaction to divalent antibodies with two needles (lower left frame, Fig. 4D). In control experiments, no antibodies were found to bind the needle of NCs isolated from the *ipaD* mutant (data not shown).

### Example 3: Anti-IpaD antibodies blocks entry of bacteria into epithelial cells

Since IpaD is required for entry of bacteria into epithelial cells [14] and since, as shown here, it is localized at the tip of the T3SA, the inventors investigated whether anti-IpaD antibodies might interfere with entry of bacteria into HeLa cells. Bacteria incubated with different concentrations of the anti-IpaD serum, or an anti-IpaB serum as a control, were used to infect HeLa cells. Exposure of bacteria to the anti-IpaD serum, but not to the anti-IpaB serum, inhibited bacterial entry in a dose-dependent manner (Fig. 5). Treatment with the anti-IpaD antibodies also inhibited entry of a *S. flexneri* 2a strain (data not shown).

### General discussion regarding Example 1 to 3

The *S. floxneri* T3SA is activated upon contact of bacteria with epithelial cells and is deregulated by inactivation of *ipaB* or *ipaD.* It was proposed that these proteins are required to form a complex pluging the T3SA. Here, the inventors present evidence that IpaD is present at the tip of the needle. Transmission electron micrograph of surface exposed needles from cross-linked bacteria showed a distinctive structure present at the tip of the needle and immunoblot analysis of mildly purified NCs indicated that IpaD is copurified with the cross-linked NCs.

Calculated averages of NCs isolated from cross-linked wild-type bacteria showed distinct densities at either sides of the needle tip. This feature was not observed in NCs isolated from both the wild-type strain that had not been treated with the cross-linker and from the *ipaD* mutant treated with the cross-linker. Results of immunoelectron microscopy indicate that the observed density at the tip of the needle contains IpaD molecules. The exact configuration of the additional density, however, cannot be retrieved from 2D projections maps. The two additional masses have dimensions of about 7 x 7 nm. Since the size of IpaD is 37-kDa, several copies of IpaD are probably present in these structures. Indeed, IpaD has been proposed to form oligomers [19] IpaD presents some functional analogies with LcrV of *Yersinia enterocolitica*, inasmuch as the two proteins have similar sizes and are both required for insertion of the proposed translocators, IpaB and IpaC in S. flexneri and YopB and UopD in *Y. enterocolitica*, in the membrane of host cells [9, 20, 21]. Recent data indicated that LcrV is localized at the tip of the T3SA needle [20]. The structure in *Yersinia* appears to be slightly different to that In *Shigella* with smaller protruding densities at the side and with a different tip.

The identification of a structural element containing IpaD at the tip of the T3SA needle provides further insights on the composition and structure of the S. *flexneri* T3SA. Very recently it was also demonstrated with biochemical methods that IpaD localizes to the T3SA needle tip, where it functions to control the secretion and proper insertion of translocators into host cell membranes [22]. The present single particle analysis, however, directly demonstrates the position of IpaD at the tip of the needle and adds credence to the hypothesis that Ipad acts as a plug to the T3SA prior to contact of bacteria with cells. As proposed for LcrV in *Yersinia*, IpaD might also facilitate insertion of components of the translocators within the cell membrane. The inhibition of entry of bacteria into HeLa cells by treatment with anti-IpaD neutralizing antibodies indicates that binding of antibodies to IpaD interferes with the function of the protein. LcrV has also been shown to be a protective antigen for plague disease in animal studies [23, 24]. Accordingly, IpaD represent an interesting target for the preparation of vaccines that would be effective against several serotypes of *Shigella.*

### Example 4: Protective effect of anti-IpaD antibodies in vivo

Intestinal iliac loops performed in the rabbit were inoculated with a suspension of 10⁹ CFU of *Shigella flexneri* serotype 5a alone or were incubated in the presence of different dilutions of a rabbit polyclonal serum specific for IpaD. The anti-IpaD polyclonal serum was produced following an immunization with a functional derivative of the IpaD protein expressed by the pMal-IpaD expression vector deposited at the CNCM on October 10, 2007 under accession number I-3839. This model summarizes the set of lesions observed during shigellosis in humans following the rupture, the invasion and the inflammatory destruction of the intestinal epithelium by this entero-invasive bacterium. These lesions are manifested through a combination of morphological alterations of the intestinal villi and edema, combined with an inflammatory cellular filtrate, in particular polynuclear neutrophils, and abscesses which eventually become ulcerated in the intestinal lumen. Overall, this gives rise to luminal mucopurulent exudates which are often invasive.

By comparing tissue destruction in the intestinal loops inoculated with bacteria alone with those having received bacteria in the presence of anti-IpaD polyclonal serum, a protective effect was observed which is dependent on the anti-IpaD antibody concentration. Indeed, when the serum used is non-diluted, no lesions are visible. However, when the serum used is in a 1/10 dilution, very discrete lesions appear, becoming clearer when a 1/100 dilution is used, all the while remaining less important than those observed with bacteria alone. No protection is observed with a control polyclonal serum directed to a non relevant protein. Therefore, the protection observed is specifically linked to the presence of anti-IpaD antibodies.

### References

[1] E.H.LaBrec, H. Schneider, T.J. Magnani, S.B. Format, Epithelial cell penetration as an essential step in the pathogenesis of bacillary dysentery. J. Bacteriol. 43 (1994) 1503-1518.
[2] C. Parsot, Shigella spp. and enteroinvasive Escherichia coli pathogenicity factors. FEMS Microbiol. Lett. 252 (2005) 11-18.
[3] F.G. van der Goot, G. Tran van Nhieu, A. Allaoui, P. Sansonetti, F. Lafont, Rafts can trigger contact-mediated secretion of bacterial effectors via a lipid-based mechanism. J Biol. Chem. 279 (2004) 47792-47798.
[4] Y. Chen, M.R. Smith, K. Thirumalai, A. Zychlinsky, A bacterial invasin induces macrophage apoptosis by binding directly to ICE. EMBO J. 15 (1996) 3853-3860.
[5] A. Kuwae, S. Yoshida, K. Tamano, H. Mimuro, T. Suzuki, C. Sasakawa, Shigella invasion of macrophage requires the insertion of IpaC into the host plasma membrane: functional analysis of IpaC. J. Biol. Chem. 276 (2001) 32230-32239.
[6] R. Ménard. M.C. Prevost. P. Gounon. P. Sansonetti. C. Dehio, The secreted Ipa complex of Shigella flexneri promotes entry into mammalian cells. Proc. Natl. Acad. Sci. USA 93 (1998)1254-1258.
[7] G. Tran Van Nhieu, R. Bourdet-Sicard. G. Dumenil, A. Blocker, P.J. Sansonetti, Bacterial signals and cell responses during Shigella entry into epithelial cells. Cell Microbiol. 2 (2000) 187-193.
[8] G. Tran Van Nhieu, E. Caron, A. Hall, P.J. Sansonetti, IpaC induces actin polymerization and filopodia formation during Shigella entry into epithelial cells. EMBO J. 18 (1999) 3249-3262.
[9] R. Ménard, P. Sansonetti, C. Parsot, The secretion of the Shigella flexneri Ipa invasins is activated by epithelial cells and controlled by IpaB and IpaD. EMBO J. 13 (1994) 5293-5302.
[10] C. Parsot, R. Menard, P. Gounon, P.J. Sansonetti, Enhanced secretion through the Shigella flexneri Mxi-Spa translocon leads to assembly of extracellular proteins into macromolecular structures. Mol. Microbiol. 16 (1995) 291-300.
[11] K.R. Turbyfill, J.A. Mertz, C.P. Mallett, E.V. Oaks, identification of epitope and surface-exposed domains of Shigella flexneri invasion plasmid antigen D (IpaD). Infect Immun. 66 (1998) 1999-2006.
[12] W.L. Picking, H. Nishioka, P.D. Heam, M.A. Baxter, A.T. Harrington, A. Blocker, W.D. Picking, IpaD of Shigella flexneri is Independently required for regulation of Ipa protein secretion and efficient insertion of IpaB and IpaC into host membranes. Infect. Immun. 73(2005) 1432-4.40.
[13] A. Allaoui, P.J. Sansonetti, C. Parsot, MxiJ, A Lipoprotein Involved in Secretion of Shigella Ipa Invasins, Is Homologous to Yscj, A Secretion Factor of the Yersinia Yop Proteins. J. Bacteriol. 174 (1992) 7661-7669.
[14] R. Menard, P.J. Sansonetti, C. Parsot, Nonpolar Mutagenesis of the Ipa Genes Defines IpaB, IpaC, and IpaD As Effectors of Shigella-Flexneri Entry Into Epithelial Cells. J. Bacteriol. 175(1993) 5899-5906.
[15] M. Sani, A. Allaoui, F. Fusetti, G.T. Oostergetel, W. Keegstra, E.J. Boekema, Structural organisation of the needle complex of the type III secretion Apparatus of Shigella flexneri. Micron (in press) 2006.
[16] N. Jouihri, M.P. Sory, A.L. Page, P. Gounon, C. Parsot, A. Allaoui, MxiK and MxiN interact with the Spa47 ATPase and are required for transit of the needle components MxiH and Mxil, but not of Ipa proteins, through the type III secretion apparatus of Shigella flexneri. Mol. Microbiol. 49 (2003) 755-767.
[17] G.T. Oostergetel, W. Keegstra, A. Brisson, Automation of specimen selection and data acquisition for protein electron crystallography. Ultramicroscopy 74 (1998) 47-59.
[18] R Schuch, A.T. Maureill, MxiM and MxiJ, base elements of the Mxi Spa type III secretion system of Shigella, interact with and stabilize the MxiD secretin in the cell envelope. J. Bacteriol. 183 (2001) 6991-6998.
[19] R. Davis, M.E. Marquart, D. Lucius, W.D. Picking, Protein-protein interactions in the assembly of Shigella flexneri invasion plasmid antigens IpaB and IpaC into protein complexes. Biochim. Biophys. Acta 1429 (1998) 45-56.
[20] C.A. Mueller, P. Broz, S.A. Muller, P. Ringler, F. Erne-Brand I., Sorg, M. Kuhn, A. Engel, G.R. Cornelis, The V-antigen of Yersinia forms a distinct structure at the tip of injectisome needles. Science 310 (2005) 674-676.
[21] L.J. Mota, Type III secretion gets an LcrV tip. Trends Microbiol. 14(2006) 197-200.
[22] M. Espina, A.J.Olive, R. Kenjale, D.S. Moore, S.F. Ausar, R.W. Kaminski, E.V. Oaks, C.R. Middaugh, W.D. Picking, W.L. Picking, IpaD localizesto the tip of the type III secretion system needle of Shigella flexneri Infect. Immun. 74 (2006) 4391-4400.
[23] J. Hill, J.E. Eyles, S.J. Elvin, G.D. Healey, R.A. Lukaszewski, R.W. Titball, Administration of antibody to the lung protects mice against pneumonic plague. Infect. Immun. 74 (2006) 3068-3070.
[24] S.J. Elvin, J.E. Eyles, K.A. Howard, E. Ravichandran, S. Somavarappu, H.O. Alpar, E.D. Williamson, Protection against bubonic and pneumonic plague with a single dose microencapsulated sub-unit vaccine. Vaccine 24 (2006) 4433-4439.

## Claims

1. A composition for use in blocking entry of at least one Shigella serotype into a permissive cell, comprising at least one of the following elements:
- an IpaD polypeptide or functional polypeptide derivative or a fragment thereof that retains the capacity of eliciting the production of anti-IpaD neutralizing antibodies against a Shigella strain when administered to an animal;
- a polynucleotide encoding an IpaD polypeptide or a fragment thereof that retains the capacity of encoding a IpaD polypeptide which elicits the production of anti-IpaD neutralizing antibodies against a Shigella strain when administered to an animal; and/or
- an anti-IpaD neutralizing antibody.

2. An IpaD polypeptide or a functional polypeptide derivative or a fragment thereof that retains the capacity of eliciting the production of anti-IpaD neutralizing antibodies against a Shigella strain when administered to an animal, or a polynucleotide encoding an IpaD polypeptide or a functional fragment thereof that retains the capacity of encoding a IpaD polypeptide which elicits the production of anti-IpaD neutralizing antibodies against a Shigella strain when administered to an animal; and/or an anti-IpaD neutralizing antibody, for use in the treatment and/or the prevention of a Shigella infection.

3. The composition or a polypeptide or a fragment thereof, a polynucleotide or a fragment thereof, or an antibody according to claim 1 or 2, wherein said IpaD polypeptide, has an amino acid sequence identical to SEQ ID NO: 1 or an amino acid sequence having at least 75% identity or at least 85% identity or at least 95% identity to SEQ ID NO:1 or said polynucleotide has a nucleotide sequence identical to SEQ ID NO: 2 or a nucleotide sequence having at least 65% identity or at least 80% identity or at least 95% identity to SEQ ID NO:2.

4. The composition or an antibody of any one of claims 1 to 3, wherein the anti-IpaD neutralizing antibody is polyclonal or monoclonal.

5. The composition of claim 1, further comprising a polyosidic antigen.

6. The composition according to claims 1, 3 or 4 or a polypeptide or a fragment thereof, or a polynucleotide or a fragment thereof, or an antibody of any one of claims 2 to 4 further comprising an adjuvant.

7. Use of at least one of the following elements for the preparation of a composition for blocking entry of at least one Shigella serotype into a permissive cell, wherein said elements are:
- an IpaD polypeptide or functional polypeptide derivative or a fragment thereof that retains the capacity of eliciting the production of anti-IpaD neutralizing antibodies against a Shigella strain when administered to an animal;
- a polynucleotide encoding an IpaD polypeptide or a fragment thereof that retains the capacity of encoding a IpaD polypeptide which elicits the production of anti-IpaD neutralizing antibodies against a Shigella strain when administered to an animal; and/or
- an anti-IpaD neutralizing antibody.

8. Use of at least one of the following elements for the preparation of a composition for the treatment and/or the prevention of a Shigella infection, wherein said elements are :
- an IpaD polypeptide or functional polypeptide derivative or a fragment thereof that retains the capacity of eliciting the production of anti-IpaD neutralizing antibodies against a Shigella strain when administered to an animal;
- a polynucleotide encoding an IpaD polypeptide or a fragment thereof that retains the capacity of encoding a IpaD polypeptide which elicits the production of anti-IpaD neutralizing antibodies against a Shigella strain when administered to an animal; and/or
- an anti-IpaD neutralizing antibody.

9. The use according to claim 7 or 8, wherein said IpaD polypeptide has an amino acid sequence identical to SEQ ID NO: 1 or an amino acid sequence having at least 75% identity or at least 85% identity or at least 95% identity to SEQ ID NO:1 or said polynucleotide has a nucleotide sequence identical to SEQ ID NO: 2 or a nucleotide sequence having at least 65% identity or at least 80% identity or at least 95% identity to SEQ ID NO:2.

10. The use of any one of claims 7 to 9, wherein the anti-IpaD neutralizing antibody is polyclonal or is monoclonal.

11. The use of any one of claims 7 to 10, further comprising a polyosidic antigen, and/or an adjuvant.

12. A kit for blocking entry of at least one Shigella serotype into a permissive cell, comprising:
- an IpaD polypeptide or functional polypeptide derivative or a fragment thereof that retains the capacity of eliciting the production of anti-IpaD neutralizing antibodies against a Shigella strain when administered to an animal;
- a polynucleotide encoding an IpaD polypeptide or a fragment thereof that retains the capacity of encoding a IpaD polypeptide which elicits the production of anti-IpaD neutralizing antibodies against a Shigella strain when administered to an animal; and/or
- an anti-IpaD neutralizing antibody.

13. An anti-IpaD neutralizing antibody for use for blocking entry of at least one Shigella serotype into a permissive cell, wherein said anti-IpaD neutralizing antibody forms an immune complex by contacting said antibody with a Shigella strain capable of infecting said permissive cell, said immune complex preventing or substantially reducing entry of said Shigella strain in the permissive cell.

14. The polypeptide or a fragment thereof, or a polynucleotide or a fragment thereof, or an antibody according to any of claims 2 to 6 for use in the preparation of a vaccine effective against several serotypes of *Shigella.*

## Patentansprüche

1. Zusammensetzung zur Verwendung im Blockieren des Eintritts von mindestens einem *Shigella* Serotyp in eine permissive Zelle, umfassend mindestens eines der folgenden Elemente:
- ein IpaD Polypeptid oder funktionelles Polypeptidderivat oder ein Fragment davon, das die Fähigkeit beibehält, die Herstellung von anti-IpaD neutralisierenden Antikörpern gegen einen *Shigella* Stamm auszulösen, wenn es an ein Tier verabreicht wird;
- ein ein IpaD Polypeptid kodierendes Polynukleotid oder ein Fragment davon, das die Fähigkeit beibehält, ein IpaD Polypeptid zu kodieren, das die Herstellung von anti-IpaD neutralisierenden Antikörpern gegen einen *Shigella* Stamm auslöst, wenn es an ein Tier verabreicht wird; und/oder
- einen anti-IpaD neutralisierenden Antikörper.

2. IpaD Polypeptid oder ein funktionelles Polypeptidderivat oder ein Fragment davon, das die Fähigkeit beibehält, die Herstellung von anti-IpaD neutralisierenden Antikörpern gegen einen *Shigella* Stamm auszulösen, wenn es an ein Tier verabreicht wird, oder ein IpaD Polypeptid kodierendes Polynukleotid oder ein funktionelles Fragment davon, das die Fähigkeit beibehält, ein IpaD Polypeptid zu kodieren, das die Herstellung von anti-IpaD neutralisierenden Antikörpern gegen einen *Shigella* Stamm auslöst, wenn es an ein Tier verabreicht wird; und/oder ein anti-IpaD neutralisierender Antikörper,
zur Verwendung in der Behandlung und/oder der Prävention einer *Shigella* Infektion.

3. Zusammensetzung oder ein Polypeptid oder ein Fragment davon, ein Polynukleotid oder ein Fragment davon, oder ein Antikörper nach Anspruch 1 oder 2, wobei das IpaD Polypeptid eine Aminosäuresequenz aufweist, die zu SEQ ID NO:1 identisch ist oder eine Aminosäuresequenz mit mindestens 75% Identität oder mindestens 85% Identität oder mindestens 95% Identität zu SEQ ID NO:1, oder das Polynukleotid eine Nukleotidsequenz aufweist, die zu SEQ ID NO:2 identisch ist oder eine Nukleotidsequenz mit mindestens 65% Identität oder mindestens 80% Identität oder mindestens 95% Identität zu SEQ ID NO:2.

4. Zusammensetzung oder Antikörper nach einem beliebigen der Ansprüche 1 bis 3, wobei der anti-IpaD neutralisierende Antikörper polyclonal oder monoclonal ist.

5. Zusammensetzung nach Anspruch 1, des Weiteren umfassend ein polyosidisches Antigen.

6. Zusammensetzung nach Ansprüchen 1, 3 oder 4 oder ein Polypeptid oder ein Fragment davon, oder ein Polynukleotid oder ein Fragment davon, oder ein Antikörper nach einem beliebigen der Ansprüche 2 bis 4, des Weiteren umfassend ein Adjuvans.

7. Verwendung von mindestens einem der folgenden Elemente zur Herstellung einer Zusammensetzung zum Blockieren des Eintritts von mindestens einem *Shigella* Serotyp in eine permissive Zelle, wobei die Elemente sind:
- ein IpaD Polypeptid oder funktionelles Polypeptidderivat oder ein Fragment davon, das die Fähigkeit beibehält, die Herstellung von anti-IpaD neutralisierenden Antikörpern gegen einen *Shigella* Stamm auszulösen, wenn es an ein Tier verabreicht wird;
- ein ein IpaD Polypeptid kodierendes Polynukleotid oder ein Fragment davon, das die Fähigkeit beibehält, ein IpaD Polypeptid zu kodieren, das die Herstellung von anti-IpaD neutralisierenden Antikörpern gegen einen *Shigella* Stamm auslöst, wenn es an ein Tier verabreicht wird; und/oder
- ein anti-IpaD neutralisierender Antikörper.

8. Verwendung von mindestens einem der folgenden Elemente zur Herstellung einer Zusammensetzung für die Behandlung und/oder die Prävention einer *Shigella* Infektion, wobei die Elemente sind:
- ein IpaD Polypeptid oder funktionelles Polypeptidderivat oder ein Fragment davon, das die Fähigkeit beibehält, die Herstellung von anti-IpaD neutralisierenden Antikörpern gegen einen *Shigella* Stamm auszulösen, wenn es an ein Tier verabreicht wird;
- ein ein IpaD Polypeptid kodierendes Polynukleotid oder ein Fragment davon, das die Fähigkeit beibehält, ein IpaD Polypeptid zu kodieren, das die Herstellung von anti-IpaD neutralisierenden Antikörpern gegen einen *Shigella* Stamm auslöst, wenn es an ein Tier verabreicht wird; und/oder
- ein anti-IpaD neutralisierender Antikörper.

9. Verwendung nach Anspruch 7 oder 8, wobei das IpaD Polypeptid eine Aminosäuresequenz aufweist, die zu SEQ ID NO:1 identisch ist oder die Polynukleotid eine Aminosäuresequenz mit mindestens 75% Identität oder mindestens 85% Identität oder mindestens 95% Identität zu SEQ ID NO:1, oder eine Nukleotidsequenz aufweist, die zu SEQ ID NO:2 identisch ist, oder eine Nukleotidsequenz mit mindestens 65% Identität oder mindestens 80% Identität oder mindestens 95% Identität zu SEQ ID NO:2.

10. Verwendung nach einem beliebigen der Ansprüche 7 bis 9, wobei der anti-IpaD neutralisierende Antikörper polyklonal oder monoklonal ist.

11. Verwendung nach einem beliebigen der Ansprüche 7 bis 10, des Weiteren umfassend ein polyosidisches Antigen und/oder ein Adjuvans.

12. Kit zum Blockieren des Eintritts von mindestens einem *Shigella* Serotyp in eine permissive Zelle, umfassend:
- ein IpaD Polypeptid oder funktionelles Polypeptidderivat oder ein Fragment davon, das die Fähigkeit beibehält, die Herstellung von anti-IpaD neutralisierenden Antikörpern gegen einen *Shigella* Stamm auszulösen, wenn es an ein Tier verabreicht wird;
- ein ein IpaD Polypeptid kodierendes Polynukleotid oder ein Fragment davon, das die Fähigkeit beibehält, ein IpaD Polypeptid zu kodieren, das die Herstellung von anti-IpaD neutralisierenden Antikörpern gegen einen *Shigella* Stamm auslöst, wenn es an ein Tier verabreicht wird; und/oder
- einen anti-IpaD neutralisierenden Antikörper.

13. Anti-IpaD neutralisierender Antikörper zur Verwendung des Blockierens des Eintritts von mindestens einem *Shigella* Serotyp in eine permissive Zelle, wobei der anti-IpaD neutralisierende Antikörper einen Immunkomplex durch Inkontaktbringen des Antikörpers mit einem *Shigella* Stamm, der zum Infizieren der permissiven Zelle fähig ist, ausbildet, wobei der Immunkomplex den Eintritt des *Shigella* Stammes in die permissive Zelle verhindert oder wesentlich reduziert.

14. Polypeptid oder ein Fragment davon, oder ein Polynukleotid oder eine Fragment davon, oder ein Antikörper nach einem beliebigen der Ansprüche 2 bis 6, zur Verwendung in der Herstellung eines Vakzins, das gegen mehrere Serotypen von *Shigella* wirksam ist.

## Revendications

1. Composition destinée à une utilisation dans le blocage de l'entrée d'au moins un sérotype de Shigella dans une cellule permissive, comprenant au moins l'un des éléments suivants :
- un polypeptide IpaD ou un dérivé polypeptidique fonctionnel ou un fragment de celui-ci qui conserve la capacité de susciter la production d'anticorps neutralisants anti-IpaD contre une souche de *Shigella* lorsqu'il est administré à un animal ;
- un polynucléotide codant pour un polypeptide IpaD ou un fragment de celui-ci qui conserve la capacité de coder pour un polypeptide IpaD qui suscite la production d'anticorps neutralisants anti-IpaD contre une souche de *Shigella* lorsqu'il est administré à un animal ; et/ou
- un anticorps neutralisant anti-IpaD.

2. Polypeptide IpaD ou un dérivé polypeptidique fonctionnel ou un fragment de celui-ci qui conserve la capacité de susciter la production d'anticorps neutralisants anti-IpaD dirigés contre une souche de *Shigella* lorsqu'il est administré à un animal, ou un polynucléotide codant pour un polypeptide IpaD ou un fragment fonctionnel de celui-ci qui conserve la capacité de coder pour un polypeptide IpaD qui suscite la production d'anticorps neutralisants anti-IpaD dirigés contre une souche de *Shigella* lorsqu'il est administré à un animal ; et/ou un anticorps neutralisant anti-IpaD,
destiné à une utilisation dans le traitement et/ou la prévention d'une infection à *Shigella.*

3. Composition ou polypeptide ou fragment de celui-ci, polynucléotide ou fragment de celui-ci, ou anticorps selon la revendication 1 ou 2, dans lequel ledit polypeptide IpaD a une séquence d'acides aminés identique à SEQ ID NO: 1 ou une séquence d'acides aminés ayant au moins 75% d'identité ou au moins 85% d'identité ou au moins 95% d'identité avec SEQ ID NO: 1 ou ledit polynucléotide a une séquence nucléotidique identique à SEQ ID NO: 2 ou une séquence nucléotidique ayant au moins 65% d'identité ou au moins 80% d'identité ou au moins 95% d'identité avec SEQ ID NO: 2.

4. Composition ou anticorps selon l'une quelconque des revendications 1 à 3, dans lequel l'anticorps neutralisant anti-IpaD est polyclonal ou monoclonal.

5. Composition selon la revendication 1, comprenant en outre un antigène polyosidique.

6. Composition selon les revendications 1, 3 ou 4 ou un polypeptide ou un fragment de celui-ci, ou un polynucléotide ou un fragment de celui-ci, ou un anticorps selon l'une quelconque des revendications 2 à 4, comprenant en outre un adjuvant.

7. Utilisation d'au moins l'un des éléments suivants pour la préparation d'une composition destinée à bloquer l'entrée d'au moins un sérotype de *Shigella* dans une cellule permissive, dans laquelle lesdits éléments sont :
- un polypeptide IpaD ou un dérivé polypeptidique fonctionnel ou un fragment de celui-ci qui conserve la capacité de susciter la production d'anticorps neutralisants anti-IpaD dirigés contre une souche de *Shigella* lorsqu'il est administré à un animal ;
- un polynucléotide codant pour un polypeptide IpaD ou un fragment de celui-ci qui conserve la capacité de coder pour un polypeptide IpaD qui suscite la production d'anticorps neutralisants anti-IpaD dirigés contre une souche de Shigella lorsqu'il est administré à un animal ; et/ou
- un anticorps neutralisant anti-IpaD.

8. Utilisation d'au moins l'un des éléments suivants pour la préparation d'une composition destinée au traitement et/ou à la prévention d'une infection à *Shigella,* dans laquelle lesdits éléments sont :
- un polypeptide IpaD ou un dérivé polypeptidique fonctionnel ou un fragment de celui-ci qui conserve la capacité de susciter la production d'anticorps neutralisants anti-IpaD dirigés contre une souche de *Shigella* lorsqu'il est administré à un animal ;
- un polynucléotide codant pour un polypeptide IpaD ou un fragment de celui-ci qui conserve la capacité de coder pour un polypeptide IpaD qui suscite la production d'anticorps neutralisants anti-IpaD dirigés contre une souche de Shigella lorsqu'il est administré à un animal ; et/ou
- un anticorps neutralisant anti-IpaD.

9. Utilisation selon la revendication 7 ou 8, dans laquelle ledit polypeptide IpaD a une séquence d'acides aminés identique à SEQ ID NO: 1 ou une séquence d'acides aminés ayant au moins 75% d'identité ou au moins 85% d'identité ou au moins 95% d'identité avec SEQ ID NO: 1 ou ladite séquence nucléotidique a une séquence nucléotidique identique à SEQ ID NO: 2 ou une séquence nucléotidique ayant au moins 65% d'identité ou au moins 80% d'identité ou au moins 95% d'identité avec SEQ ID NO: 2.

10. Utilisation selon l'une quelconque des revendications 7 à 9, dans laquelle l'anticorps neutralisant anti-IpaD est polyclonal ou est monoclonal.

11. Utilisation selon l'une quelconque des revendications 7 à 10, comprenant en outre un antigène polyosidique, et/ou un adjuvant.

12. Trousse pour bloquer l'entrée d'au moins un sérotype de *Shigella* dans une cellule permissive, comprenant :
- un polypeptide IpaD ou un dérivé polypeptidique fonctionnel ou un fragment de celui-ci qui conserve la capacité de susciter la production d'anticorps neutralisants anti-IpaD dirigés contre une souche de *Shigella* lorsqu'il est administré à un animal ;
- un polynucleotide codant pour un polypeptide IpaD ou un fragment de celui-ci qui conserve la capacité de coder pour un polypeptide IpaD qui suscite la production d'anticorps neutralisants anti-IpaD dirigés contre une souche de *Shigella* lorsqu'il est administré à un animal ; et/ou
- un anticorps neutralisant anti-IpaD.

13. Anticorps neutralisant anti-IpaD destiné à une utilisation pour bloquer l'entrée d'au moins un sérotype de *Shigella* dans une cellule permissive, dans lequel ledit anticorps neutralisant anti-IpaD forme un complexe immun par la mise en contact dudit anticorps avec une souche de *Shigella* capable d'infecter ladite cellule permissive, ledit complexe immun empêchant ou réduisant sensiblement l'entrée de ladite souche de *Shigella* dans la cellule permissive.

14. Polypeptide ou fragment de celui-ci, ou polynucléotide ou fragment de celui-ci, ou anticorps selon l'une quelconque des revendications 2 à 6 destiné à une utilisation dans la préparation d'un vaccin efficace contre plusieurs sérotypes de *Shigella.*
